# EUROPÄISCHE PATENTSCHRIFT

(11) **EP 3 429 520 B1**
(45) Veröffentlichungstag und Bekanntmachung des Hinweises auf die Patenterteilung: **29.04.2020**
(21) Anmeldenummer: 17713451.7
(22) Anmeldetag: 13.03.2017
(51) Int. Cl.: A61F 9/013, A61F 2/14

(54) **VORRICHTUNG UND VERFAHREN ZUM GEWINNEN EINES SCHICHT-TRANSPLANTATS DER MENSCHLICHEN AUGENHORNHAUT**
DEVICE AND METHOD FOR OBTAINING A LAYERED TRANSPLANT OF THE HUMAN CORNEA
DISPOSITIF ET PROCÉDÉ POUR OBTENIR UN GREFFE DE COUCHE DE CORNEE HUMAINE

(30) Priorität: 14.03.2016 DE 102016104680
(43) Veröffentlichungstag der Anmeldung: 23.01.2019
(73) Patentinhaber: Vossamed GmbH & Co. KG, 50667 Köln (DE)
(72) Erfinder: GERTEN, Georg, 53121 Bonn (DE)
(74) Vertreter: Hennicke, Ernst Rüdiger
(86) Internationale Anmeldenummer: PCT/IB2017/051439
(87) Internationale Veröffentlichungsnummer: WO 2017/158490

(56) Entgegenhaltungen:
- WO-A1-2015/111040
- GB-A- 2 448 170
- US-A- 4 077 411
- US-A1- 2004 243 159
- US-B2- 7 147 648
- KUMAR DHIVYA ASHOK ET AL: "Postoperative spectral-domain optical coherence tomography evaluation of pre-Descemet endothelial keratoplasty grafts", JOURNAL CATARACT AND REFRACTIVE SURGERY, SURGERY, FAIRFAX, VA, US, Bd. 41, Nr. 7, 15. August 2015 (2015-08-15), Seiten 1535-1536, XP029260202, ISSN: 0886-3350, DOI: 10.1016/J.JCRS.2015.05.015

## Beschreibung

Die Erfindung betrifft eine Vorrichtung und ein Verfahren zum Gewinnen eines Schicht-Transplantats der menschlichen Augenhornhaut (Cornea) aus einer Corneo Skleralscheibe mittels Flüssigkeits- oder Gas-Injektion, womit insbesondere die inneren Cornea Schichten präpariert werden können.

In den letzten Jahren ist es zu einem Paradigmenwechsel bei der Transplantationschirurgie der menschlichen Cornea gekommen. Bei Hornhauterkrankungen, die eine Transplantation erforderlich machten, wurde früher standardgemäß die Cornea des erkrankten Auges im Wesentlichen vollständig durch eine menschliche Spendercornea ersetzt. Dazu wurde die erkrankte Patientenhornhaut in ganzer Dicke ausgestanzt und durch eine Spendercornea eines toten Menschen ersetzt. Seit etwa zehn Jahren versucht man hingegen zunehmend erfolgreich, erkrankte Cornea nicht mehr in ganzer Dicke auszustanzen und zu transplantieren, sondern gezielt nur noch erkrankte, insbesondere die inneren Schichten der Hornhaut zu ersetzen, also die auch als Endothelschicht bezeichnete Descemet Membran. Hierzu müssen zu ersetzenden Schichten entsprechend schichtweise auch aus der Spendercornea gewonnen werden.

Dies geschieht derzeit meist noch manuell. Zunächst wird aus dem Leichenauge eine sogenannte Corneo-Skleralscheibe gewonnen, aus der dann wiederum einzelne Schichten präpariert werden.

Speziell zur Präparation der inneren Schichten der Cornea (Endothel Transplantation DMEK - Descemet Membrane Endothelial Keratoplasty) muss aus der Corneo Skleralscheibe zunächst die innere Endothelschicht mit ihrer Membran (Descemet Membran) gewonnen werden. Dazu wird diese üblicherweise mit einem Mikrospatel/Messer innen an der Corneo-Skleralscheibe ab präpariert. Dies ist zeitaufwendig und fehleranfällig. Die Endothelschicht muss dazu notwendigerweise berührt werden, was einen Verlust von Endothelzellen zur Folge hat. Diese weitgehend manuelle Präparation ist fehleranfällig und sehr vom Geschick und der Erfahrung des präparierenden Arztes abhängig.

Es ist daher auch schon vorgeschlagen worden (vgl. zB. https://en.wikipedia.org/wiki/PreDescemet's_Endothelial_Keratoplasty #/media/File:It describes Pdek eye surgery.jpg), ein Gas oder eine Flüssigkeit zwischen die einzelnen Schichten der Decemet'schen Membran oder zwischen Descemet und Stroma zu injizieren und hierdurch die betreffenden Schichten voneinander zu trennen. Dazu wird in einer Vorpräparation z.B. mit einer Mikrochirurgischen Lanze definierter Grösse (ca 0,5-2,5mm) eine Öffnung in der Corneo-Skleralscheibe etwa am Übergang zwischen Lederhaut und Hornhaut geschaffen, durch die dann eine feine Mikrochirurgische Hohlnadel (ca 0,5-2,5mm) zwischen Stroma und Descemet Membran plaziert werden kann. Über die Hohlnadel kann Flüssigkeit/Gas zwischen die Einzelschichten der Cornea gepumpt werden, um diese Schichten trennt. Beim Injizieren der Flüssigkeit/ Gases werden jedoch Schwachstellen der Decemet'schen Membran (z Bsp bei vorangegangener Kataraktoperation beim noch lebenden Spender) zum Problem, da diese dann durch den Druck der injizierten Flüssigkeit aufplatzen und es zum Einreißen des Transplantates mit Verlust von Flüssigkeit kommen kann. Weiterhin kann Gas/Flüssigkeit an der Hohlnadel vorbei durch die vorpräparierte Öffnung vorbei zurück nach außen fließen. Auch andere Lecks oder Schwachstellen können sich insbesondere in den Aussenbereichen der Membran nach auftun, durch die hindurch die Flüssigkeit abfließen kann, so dass eine zuverlässig vollständige Separation der Schichten voneinander nur schwer erreichbar ist. Das Dokument WO 2015/111040 offenbart Vorrichtung und Verfahren zum Gewinnen eines Schicht-Transplantats der menschlichen Augenhornhaut mittels Flüssigkeits-Injektion, mit einer Halteeinrichtung für die Skleralscheibe.

Aufgabe der Erfindung ist es, eine Vorrichtung und ein Verfahren der eingangs genannten Art zu schaffen, womit ein Schicht-Transplantat der menschlichen Augenhornhaut (Cornea) aus einer Corneo Skleralscheibe mittels Flüssigkeits- oder Gas-Injektion einfacher und zuverlässiger gewonnen werden kann.

Diese Aufgabe wird mit der Erfindung durch eine Vorrichtung gemäß Anspruch 1 und ein Verfahren gemäß Anspruch 11 gelöst.

Mit der Erfindung kann die innere Descemet Membran zuverlässig und berührungsfrei durch Injizieren einer Flüssigkeit oder eines Gases von Stroma der Cornea abgehoben werden kann, ohne dass es zu Einrissen, Rückfluss von Flüssigkeit/Gas oder Transplantateinrissen an Schwachstellen kommt.

Dies wird mittels des elastischen Dichtelements der Vorrichtung erreicht, das sich vorzugsweise in Form einer Ringstruktur an die eine Seite (insbesondere die Innenseite) der Cornea anlegt und die in die Skleralscheibe eingeschobene Hohlnadel gegenüber der Skleralscheibe zuverlässig abdichtet. Hierdurch ist sichergestellt, dass die Flüssigkeit/Gas unter die Descemetsche Membran gelangt und nicht an der Hohlnadel vorbei zurückfließen kann. Da die Skleralscheibe zuverlässig von der Halteeinrichtung gehalten wird, beispielsweise, indem sie in einer bevorzugten Ausführungsform zwischen zwei Spannelementen eigespannt ist, während die Flüssigkeit/das Gas injiziert wird, kann das verwendete Fluid auch nicht durch andere Lecks oder Schwachstellen an den Außenbereichen der Membran nach außen abfließen. Nach dem derart durchgeführten Trennen der Schichten voneinander kann die abgehobene Descemet Endothelschicht dann ausgestanzt und als singuläre Schicht weiter transplantiert werden.

In vorteilhafter Ausgestaltung der Erfindung weist die Halteeinrichtung ein erstes ringförmigen Spannelement und mindestens ein mit dem ersten Spannelement zusammenwirkendes, zweites Spannelement auf, zwischen welchen Spannelementen die Skleralscheibe an einem vorzugsweise ringförmigen äußeren Haltebereich einspannbar ist. Es ist auch möglich, dass die Halteeinrichtung eine Unterdruckanordnung aufweist, mittels der die Skleralscheibe an eine Haltefläche ansaugbar ist. Die Haltefläche oder ein Teil von dieser kann beispielsweise kalottenartig oder konkav vertieft und somit an die konvexe Außenkontur der zu haltenden Skleralscheibe angepasst sein.

Besonders vorteilhaft ist es, wenn das erste Spannelement am Randbereich einer etwa kalottenartigen Vertiefung eines Haltebechers für die Skleralscheibe ausgebildet ist. Zur Präparation kann die vorpräparierte Skleralscheibe so in den Haltebecher eingelegt werden, dass die Hornhaut mit ihrer äußeren Epithelschicht an der kalottenartige Vertiefung anliegt, während die innere Endothelschicht mit der diese tragenden Descemtmembran oben frei zugänglich zur Präparation liegt. Als Haltebereich ist an der Skleralscheibe regelmäßig ein ringförmiger Abschnitt der Lederhaut des vorpräparierten Spenderauges belassen worden, der dann auf dem Randbereich der kalottenartigen Vertiefung zur Auflage kommt. Wenn eine Unterdruckanordnung vorgesehen ist, können Ansaugöffnungen in der kalottenartigen Vertiefung und/oder an deren Randbereich vorgesehen sein, durch die die Skleralscheibe dann angesaugt wird.

Das zweite Spannelement kann bevorzugt als Spannring mit einer die Zugangszone bildenden Unterbrechung ausgebildet sein. In weiter vorteilhafter Ausgestaltung der Erfindung ist es möglich, dass das erste und das zweite Spannelement schwenkbar und im Spannzustand verriegelbar miteinander verbunden sind.

Das Dichtelement kann vorzugsweise im Wesentlichen aus einem kompressiblen Material und/oder einem aufblasbaren Schlauchelement bestehen. Besonders bevorzugt ist es, dass sich das Dichtelement zumindest im Wesentlichen über den gesamten Umfang der Spanneinrichtung erstreckt, also nicht nur die Zugangszone abdeckt, sondern die eingespannte Skleralscheibe im Wesentlichen vollumfänglich abdichtet, so dass jedenfalls das mindestens eine zweite Spannelement kein Abdichtfunktion erfüllen muss.

Es ist möglich, ein Druckelement vorzusehen, das unter Ausbildung eines Abstands mit wenigstens einem der ersten und zweiten Spannelemente verbunden ist und als Widerlager für das Dichtelement dient. Dieses Druckelement kann ähnlich wie eines der Spannelement ausgestaltet sein, beispielsweise als Ring aus einem im Wesentlichen starren Material, der mittels eines oder mehrerer Haltebügel(s) am Haltebecher o.dgl. im Abstand von der Spanneinrichtung gehaltert werden kann. Beispielsweise ist eine verschwenk- und verriegelbare Anordnung zweckmäßig, derart, dass nach dem Zuschwenken und Verriegeln des Druckelements zwischen diesem und der Spanneinrichtung bzw. der damit gehaltenen Skleralscheibe das Abdichtelement angeordnet ist, das dann ggf. noch aufgepumpt werden kann, um die erwünschte Abdichtung insbesondere im Bereich des Zugangs zu bewirken. Es ist auch möglich, das Druckelement von oben auf die Halteeinrichtung aufzustecken und an diesem, z.B. mit einem Bajonettverschluss zu verriegeln.

In ganz besonders vorteilhafter Weiterbildung der Erfindung kann ein vorzugsweise an der Spanneinrichtung insbesondere konzentrisch zu dem/den ringförmigen Spannelement(en) bzw. der Halteeinrichtung angeordnetes Messerelement () vorgesehen sein, das in Schneidkontakt mit der Descemet Membran der eingespannten Sklerealscheibe () bringbar ist. Das Messerelement kann z.B. an dem Druckelement angeordnet sein, insbesondere derart, dass die Descemet Membran, die sich beim Injizieren von Flüssigkeit in die Skleralscheibe von der Stromaschicht abhebt, aufgrund dieser Positionsänderung automatisch in Kontakt mit dem Messerelement kommt und somit entsprechend automatisch ausgeschnitten wird. Hierzu ist es besonders vorteilhaft, wenn das Messerelement als an die gewünschte Form des zu gewinnenden Schicht-Transplantats angepasstes, vorzugsweise kreisringförmiges Stanzmesser ausgestaltet ist.

Mit der Erfindung werden also eine Vorrichtung und ein Verfahren zum Erzeugen von Schicht-Transplantaten der menschlichen Augenhornhaut (Cornea), speziell der inneren Cornea Schichten geschaffen, womit auf besonders einfache und elegante Weise die innerste, dünne (ca. 5-20µm) Schicht der Cornea (Descemet/Endothel Schicht) von der Cornea Stroma Schicht getrennt werden kann. Dies geschieht mittels einer Flüssigkeit oder eines Gases, das/die zwischen diese beiden Schichten injiziert wird. Dazu wird eine sogenannte Corneo-Skleralscheibe benutzt, die üblicher Weise zur Transplantation der Hornhaut aus einem menschlichen Auge eines Verstorbenen entnommen wird. Die Corneo Skleralscheibe wird in der Vorrichtung eingespannt und hierdurch fixiert. Nach einer Vorpräparation einer Öffnung wird durch diese eine feine mikrochirurgische Hohlnadel (ca. 0,5-2,5mm) zwischen Stroma und Descemet Membran platziert. An die Hohlnadel ist ist ein Schlauchsystem angeschlossen, durch das Flüssigkeit oder ein Gas unter geeignetem Druck zwischen die Einzelschichten der Cornea geleitet werden kann und damit diese Schichten trennt. Dabei ist an der zumindest in dem Bereich der Cornea, in dem die Hohlnadel zwischen die Schichten eingeführt ist, mittels des elastischen Dichtelements eine zuverlässige Abdichtung der Hohlnadel gegenüber der Skleralscheibe sichergestellt. Das Dichtelement kann dabei beispielsweise mit einem Widerlager z.B. in Form einer Ringstruktur gegen die Skleralscheibe gedrückt und fixiert sein. Der erfindungsgemäße Aufbau stellt sicher, dass die Flüssigkeit/das Gas im Wesentlichen vollständig unter die Descemetsche Membran gelangt und nicht an der Hohlnadel vorbei oder durch andere Lecks oder Schwachstellen der Außenbereiche der Membran nach außen abfließt. Falls Flüssigkeit verwendet wird, kann diese auch schon Farbstoffe enthalten (z b Trypan Blau 0,05%), die eine Färbung des Transplantates ermöglichen. Nach Injektion der Flüssigkeit/Gas ist die Decemet'sche Membran mit der aufliegenden Endothelschicht vom Stroma getrennt. Das Transplantat kann dann weiter berarbeitet werden, z.B. indem die von der Stromaschicht getrennte Decemet'schen Membran auf die den gewünschten Durchmesser (ca. 7-9.5mm) kreisrund ausgestanzt wird. Dann entsteht eine dünne Rolle aus Decemet'sche Membran mit der aufliegender Endothelschicht, die zum Einführen in eine Patientenauge geeignet ist.

Weitere Merkmale und Vorteile der Erfindung ergeben sich aus der nachfolgenden Beschreibung und der Zeichnung, worin eine bevorzugte Ausführungsform der Erfindung anhand eines Beispiels näher dargestellt ist. Es zeigt:

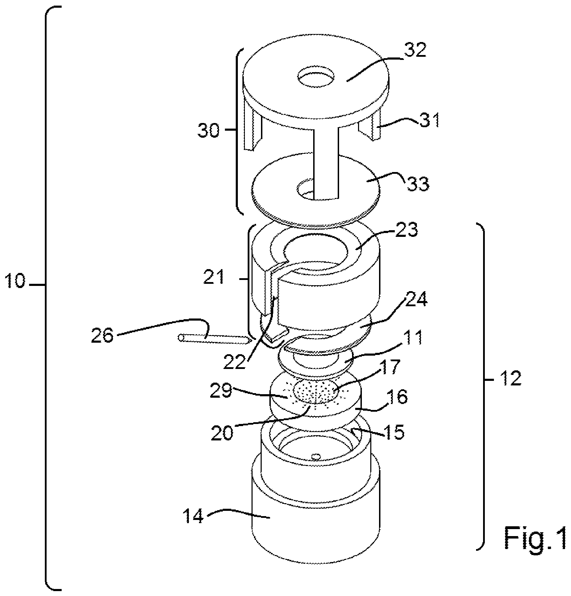

- Fig.1: ein Ausführungsbeispiel einer erfindungsgemäßen Vorrichtung in einer perspektivischen, auseinandergezogenen Darstellung ihrer einzelnen Bestandteile von schräg oben;
- Fig.2: den Gegenstand der Fig. 1 in einem Vertikalschnitt;
- Fig.3: die Vorrichtung gemäß Fig. 1 und Fig. 2 im zusammengebauten Zustand, ebenfalls im Vertikalschnitt; und

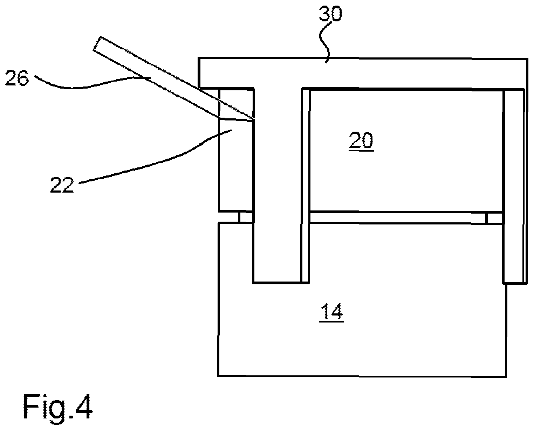

- Fig.4: den Gegenstand der Fig. 3 in einer Seitenansicht.

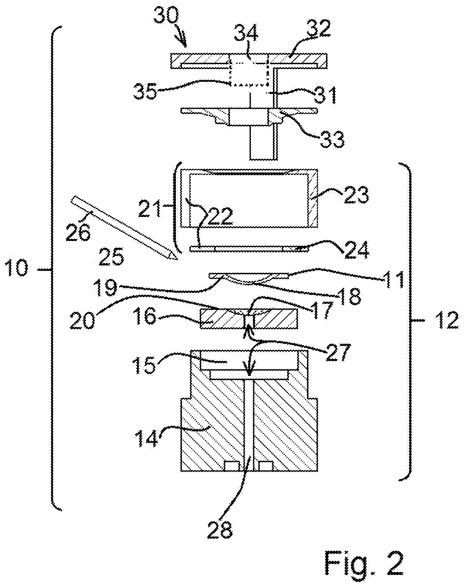

Die in der Zeichnung dargestellte und in ihrer Gesamtheit mit 10 bezeichnete Vorrichtung dient dazu, einem Arzt die Gewinnung eines Schicht-Transplantats der menschlichen Augenhornhaut aus einer Corneo-Skleralsscheibe zu erleichtern, die in Fig. 2 am besten erkennbar und mit 11 bezeichnet ist.

Die Vorrichtung hat eine Halteeinrichtung 12, um die Skleralscheibe 11. während der Präparation zuverlässig zu halten. Hierfür weist die Vorrichtung ein unteres Sockelteil 14 mit einer zylindrischen Aufnahme 15 in der oberen Stirnseite auf, in die ein Einsatz 16 mit einer kalottenartigen, konkav eingezogene Vertiefung 17 eingesetzt ist. Die Vertiefung 17 ist an die etwa konvexe Form der aus einem Auge vorpräparierten, die Hornhaut 18 und einen ringförmigen Abschnitt 19 der Lederhaut des Spenderauges aufweisenden Skleralscheibe 11 angepasst. Der die Vertiefung 17 umfangsseitig begrenzende Randbereich des bündig mit der oberen Stirnseite liegenden Einsatzes 16 bildet ein erstes Spannelement 20, auf dem der ringförmige Abschnitt 19 der Lederhaut der Skleralscheibe 11 aufliegt.

Oberhalb der Stirnseite ist ein an die darunter liegende Ringform des ersten Spannelements 20 angepasstes, zweites Spannelement 21 angeordnet, das allerdings in einer Zugangszone 22 unterbrochen ist, beispielsweise über einen Umfangsbereich von 10 bis 40°. Das zweite Spannelement 21 besteht im Wesentlichen aus einer von oben auf das Sockelteil 14 aufsetzbaren Drucktasse 23 mit einer darin bevorzugt austauschbar eingesetzten Druckscheibe 24, wobei Drucktasse 23 und Druckscheibe 24 im jeweils selben Umfangsbereich über einen Umfangsabschnitt von vorzugsweise 10 bis 40° unterbrochen sind, um die Zugangszone 22 zu schaffen. Das aus Drucktasse und Druckscheibe bestehende, zweite Spannelement 22 ist gegen das erste Spannelement 20 anstellbar, wodurch der ringförmige Randbereich 18 der Skleralscheibe, der im Wesentlichen von Lederhaut gebildet wird, über den größten Teil seines Umfangs zwischen den beiden Spannelementen eingespannt werden kann, jedoch in der Zugangszone 22 von der oben liegenden Augeninnenseite 23 für den Präparator zugänglich bleibt. Dieser kann somit im Bereich der Zugangszone 22 von oben eine Öffnung in der Skleralscheibe erzeugen und eine Hohlnadel 26 zwischen Descemet Membran und Stroma der Cornea in der Skleralscheibe einführen.

Das Sockelteil 14 sowie der darin eingesetzte Einsatz 16 sind zur zusätzlichen Fixierung der Skleralscheibe auch in deren zentralen, im Wesentlichen von der Hornhaut des Spenderauges gebildeten Mittelteil mit einer Unterdruckeinrichtung 27 in Form einer Vakuumleitung 28 sowie einer Vielzahl von in die kalottenartige Vertiefung 17 und den Randbereich 20 des Einsatzes 16 mündenden Ansaugöffnungen 29 versehen, die mit der Vakuumleitung in Verbindung sind und bei Betrieb eines angeschlossenen Unterdruckerzeugers (nicht dargestellt) an der Kalottenfläche und dem Randbereich einen Unterdruck erzeugen , so dass so die Hornhaut 18 in der Vertiefung 17 und der Lederhautrand 19 am Randbereich 20 angesaugt und so durch den sich zwischen ihrer Ober-und Unterseite einstellenden Druckunterschied gehalten wird.

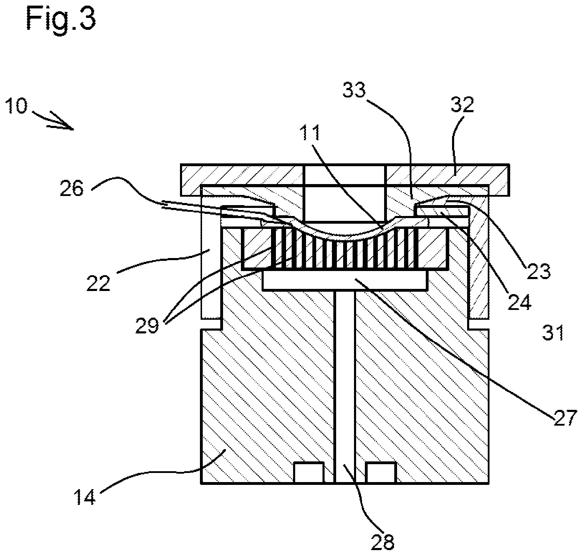

Nachdem der Präparator die Hohlnadel an der gewünschten Stelle der Zugangszone 22 und in die gewünschte Tiefe zwischen die Descemet-Membran und die Stroma der Hornhaut eingeführt hat, wird von oben auf die Drucktasse 23 und das Sockelteil 15 ein etwa hockerartiger Aufsatz 30 aufgeschoben, der drei gleichmäßig über den Umfang verteilt angeordnet Führungsbeine 31 und ein oberes, ringförmiges Druckelement 32 aufweist, das als Widerlager für ein an seiner Unterseite (Fig.3) montiertes, ebenfalls ringförmiges, elastisches Dichtelement 33 dient. Das Dichtelement 33 ist bei dem gezeigten Ausführungsbeispiel als beispielsweise im FiPFG- Verfahren (Formed in place Foamed Gasket) angeschäumter, weichelastischer Dichtkörper aus einem PUR-Schaummaterial ausgebildet. Es ist auch möglich, ihn als Schlauchkörper auszuführen, der mit einem Gas befüllt werden kann. Wenn der Aufsatz 30 mit dem Dichtelement 33 mit seinen Führungsbeinen vollständig auf die Drucktasse 23 und das Sockelteil 15 aufgeschoben ist, dass sich die, verformt sich das Dichtelement 33 elastisch und liegt von oben so an der Halteeinrichtung 12 und der darin aufgenommenen Skleralscheibe 11 an, dass diese auch in der Zugangszone 22 von oben beaufschlagt wird, so dass die zuvor dort eingeführte Hohlnadel 26 in der Öffnung, durch die sie in die Skleralscheibe 11 unter die Descemet Membran eingeführt ist, abgedichtet wird. Es ist dann möglich, eine Flüssigkeit oder ein Gas durch die Hohlnadel 26 in die Skleralscheibe unter Druck zu injizieren, wobei die von dem Dichtelement 33 bewirkte Abdichtung verhindert, dass das injizierte Fluid aus der Öffnung zurück fließen kann. Die injizierte Flüssigkeit trennt die Descemet Membram von der Stromaschicht der Cornea und hebt sie entgegen der Wölbung der Hornhaut nach oben an, so dass sie leicht frei präpariert werden kann. Da die mit dem Dichtelement bewirkte Abdichtung über den gesamten Umfang der Skleralscheibe erfolgt, können auch dort möglicherweise befindliche Schwachstellen in der Cornea nicht aufplatzen und einen unerwünschten Fluidauslass ermöglichen.

Es ist möglich, vorzugsweise an dem Aufsatz 30 in der in diesem am Druckelement 32 vorgesehenen ringförmigen Öffnung 34 ein ringförmiges Messerelement (strichpunktiert bei 35 in Fig.2 angedeutet) vorzusehen, dessen Klinge nach unten in Richtung auf die eingespannte Skleralsscheibe vorsteht. Beim Injizieren des Fluids in die Skleralscheibe und dem damit bewirkten Aufwölben der Descemet Membran nach oben kommt diese in Kontakt mit der Messerklinge 36 und wird von dieser automatisch in der gewünschten Form und Größe ausgeschnitten und steht somit für die Implatation im Auge des Empfängers zur Verfügung.

## Patentansprüche

1. Vorrichtung zum Gewinnen eines Schicht-Transplantats der menschlichen Augenhornhaut (Cornea) aus einer Corneo Skleralscheibe mittels Flüssigkeits- oder Gas-Injektion, mit
- einer Halteeinrichtung (12) für die Skleralscheibe (11); **gekennzeichnet durch**:
- mindestens einer an der Halteeinrichtung (12) ausgebildeten Zugangszone (22) zu der in dieser gehaltenen Skleralscheibe (11), in der eine Hohlnadel (26) im Randbereich der Cornea (18) durch eine Öffnung in der Skleralscheibe (11) zwischen Descemet Membran und Stroma der Cornea einführbar ist; und
- einem wenigstens in der Zugangszone (22) gegen die Halteeinrichtung (12) bzw. die darin gehaltene Skleralscheibe (11) anstellbaren, elastischen Dichtelement (33) zur abdichtenden Einspannung der Skleralscheibe (11) und der durch die Öffnung eingeführten Hohlnadel (26) zumindest im Bereich der Zugangszone (22).

2. Vorrichtung nach Anspruch 1, **dadurch gekennzeichnet, dass** die Halteeinrichtung (12) ein erstes ringförmiges Spannelement (20) und mindestens ein mit dem ersten Spannelement (20) zusammenwirkendes, zweites Spannelement (21) aufweist, zwischen welchen Spannelementen (20,21) die Skleralscheibe (11) an einem vorzugsweise ringförmigen äußeren Haltebereich (19) einspannbar ist.

3. Vorrichtung nach Anspruch 1 oder 2, **dadurch gekennzeichnet, dass** die Halteeinrichtung (12) eine Unterdruckanordnung (27) aufweist, mittels der die Skleralscheibe (11) an eine Haltefläche (17, 20) ansaugbar ist.

4. Vorrichtung nach Anspruch 2 oder 3, **dadurch gekennzeichnet, dass** das erste Spannelement (20) am Randbereich einer etwa kalottenartigen Vertiefung (17) eines einsatzes (16) für die Skleralscheibe (11) ausgebildet ist.

5. Vorrichtung nach einem der Ansprüche 2 bis 4, **dadurch gekennzeichnet, dass** das zweite Spannelement (21) als Spannring oder Drucktasse (23) mit eingesetzter Druckscheibe (24) ausgebildet ist, worin umfangsseitig eine die Zugangszone (22) bildende Unterbrechung vorgesehen ist.

6. Vorrichtung nach einem der Ansprüche 2 bis 5, **dadurch gekennzeichnet, dass** das erste und das zweite Spannelement schwenkbar und im Spannzustand verriegelbar miteinander verbunden sind.

7. Vorrichtung nach einem der Ansprüche 1 bis 6, **dadurch gekennzeichnet, dass** das Dichtelement (33) im Wesentlichen aus einem kompressiblen Material und/oder einem aufblasbaren Schlauchelement besteht.

8. Vorrichtung nach einem der Ansprüche 1 bis 7, **gekennzeichnet durch** ein Druckelement, das unter Ausbildung eines Abstands mit wenigstens einem der ersten und zweiten Spannelemente verbunden ist und als Widerlager für das Dichtelement dient.

9. Vorrichtung nach einem der Ansprüche 1 bis 8, **gekennzeichnet durch** ein insbesondere konzentrisch zu dem/den ringförmigen Spannelement(en) (20;21) angeordnetes Messerelement (35), das in Schneidkontakt mit der Descemet Membran der eingespannten Sklerealscheibe (11) bringbar ist.

10. Vorrichtung nach Anspruch 9, **dadurch gekennzeichnet, dass** das Messerelement (35) als an die gewünschte Form des zu gewinnenden Schicht-Transplantats angepasstes, vorzugsweise kreisringförmiges Stanzmesser ausgestaltet ist.

11. Verfahren zum Gewinnen eines Schicht-Transplantats der menschlichen Augenhornhaut (Cornea) aus einer Corneo Skleralscheibe eines toten Spenders mittels Flüssigkeits- oder Gas-Injektion, unter Verwendung einer Vorrichtung nach einem der Ansprüche 1 bis 10, mit den Verfahrensschritten:
- Halten der aus einem Spenderauge vorpräparierten Skleralscheibe (11) mittels einer Halteeinrichtung (12) vorzugsweise an einem ringförmigen äußeren Haltebereich (19) derart, dass an oder neben der Halteeinrichtung (12) mindestens eine Zugangszone (22) zu der eingespannten Skleralscheibe ausgebildet ist;
- Erzeugen einer Öffnung in der Skleralscheibe im Bereich der Zugangszone (22) und Einführen einer Hohlnadel (26) zwischen Descemet Membran und Stroma der Cornea in der Skleralscheibe (11) ;
- Abdichten der Öffnung gegenüber der in sie eingeführten Hohlnadel (26) mittels eines elastischen Dichtelements (33), das wenigstens im Bereich der Zugangszone (22) gegen die Halteeinrichtung bzw. die darin gehaltene Skleralscheibe angestellt wird und dabei die Skleralsscheibe und die darin eingeführte Hohlnadel im Bereich der Zugangszone zwischen sich und der Halteeinrichtung (12) abdichtend einspannt;
- Einleiten von Flüssigkeit oder Gas durch die Hohlnadel unter geeignetem Überdruck zwischen die Descemet Membran und die Stroma der Cornea in der Skleral-scheibe (11); und
- Freischneiden der derart von der Stromaschicht der Cornea getrennten Descemet Membran.

12. Verfahren nach Anspruch 11, **dadurch gekennzeichnet, dass** das Freischneiden der Descemet Membran mittels eines vorzugsweise ringförmigen Stanzmessers (35) erfolgt, das an der Vorrichtung vorzugsweise konzentrisch zu der Halteeinrichtung (12) angeordnet ist und das beim Einleiten der Flüssigkeit oder des Gases in die Skleralscheibe in Schneidkontakt mit der sich von der Stromaschicht lösenden Descemet Membran gelangt.

13. Verfahren nach Anspruch 11 oder 12, **dadurch gekennzeichnet, dass** das Dichtelement (33) einen elastischen Schlauch aufweist oder aus einem solchen besteht, der zur Abdichtung mittels eines Gases, vorzugsweise Druckluft aufgepumpt wird.

## Claims

1. Device for obtaining a layered transplant of the human cornea from a corneoscleral disc by means of liquid or gas injection, comprising
- a holding device (12) for the scleral disc (11); **characterised by**
- on the holding device (12) at least one access zone (22) to the scleral disc (11) held therein, in which a hollow needle (26) can be inserted into the edge region of the cornea (18) through an opening in the scleral disc (11) between Descemet membrane and stroma of the cornea; and
- an elastic sealing element (33) which at least in the access zone (22) can be set against the holding device (12) or the scleral disc (11) held therein for sealingly clamping the scleral disc (11) and the hollow needle (26) inserted through the opening, at least in the region of the access zone (22).

2. Device according to claim 1, **characterised in that** the holding device (12) has a first annular clamping element (20) and at least one second clamping element (21) cooperating with the first clamping element (20), between which clamping elements (20, 21) the scleral disc (11) can be clamped on a preferably annular outer holding region (19).

3. Device according to claim 1 or 2, **characterised in that** the holding device (12) has a vacuum arrangement (27) by means of which the scleral disc (11) can be sucked onto a holding surface (17, 20).

4. Device according to claim 2 or 3, **characterised in that** the first clamping element (20) is formed at the edge region of an approximately dome-like depression (17) of an insert (16) for the scleral disc (11).

5. Device according to any one of claims 2 to 4, **characterised in that** the second clamping element (21) is formed as a clamping ring or pressure cup (23) with an inserted pressure disc (24), an interruption forming the access zone (22) being provided at the periphery.

6. Device according to any one of claims 2 to 5, **characterised in that** the first and the second clamping element are pivotably connected to each other and are lockable in the clamping state.

7. Device according to any one of claims 1 to 6, **characterised in that** the sealing element (33) consists essentially of a compressible material and/or an inflatable tube element.

8. Device according to any one of claims 1 to 7, **characterised by** a pressure element which is connected to at least one of the first and second clamping elements while forming a spacing and serves as an abutment for the sealing element.

9. Device according to any one of claims 1 to 8, **characterised by** a knife element (35) arranged in particular concentrically to the annular clamping element(s) (20; 21), which knife element can be brought into cutting contact with the Descemet membrane of the clamped scleral disc (11).

10. Device according to claim 9, **characterised in that** the knife element (35) is configured as a punching knife with a form, preferably annular, adapted to the desired shape of the layered transplant to be obtained.

11. Method for obtaining a layered transplant of the human cornea from a corneoscleral disc of a dead donor by means of liquid or gas injection, using a device according to any one of claims 1 to 10, comprising the steps of:
- holding the scleral disc (11), pre-prepared from a donor eye, by means of a holding device (12), preferably on an annular outer holding region (19), in such a way that at least one access zone (22) to the clamped scleral disc is formed on or next to the holding device (12);
- producing an opening in the scleral disc in the region of the access zone (22) and introducing a hollow needle (26) into the scleral disc (11) between Descemet membrane and stroma of the cornea ;
- sealing the opening with respect to the hollow needle (26) inserted therein by means of an elastic sealing element (33), which is positioned against the holding device or the scleral disc held therein at least in the region of the access zone (22) and thereby sealingly clamps the scleral disc and the hollow needle inserted therein between itself and the holding device (12) in the region of the access zone;
- introducing liquid or gas through the hollow needle under suitable overpressure between the Descemet membrane and the stroma of the cornea in the scleral disc (11); and
- cutting out the Descemet membrane thus separated from the stroma layer of the cornea.

12. Method according to claim 11, **characterised in that** the Descemet membrane is cut out by means of a punching knife (35), which is preferably annular and which is arranged on the device, preferably concentrically to the holding device (12), and which, when the liquid or the gas is introduced into the scleral disc, comes into cutting contact with the Descemet membrane that is detaching itself from the stroma layer.

13. Method according to claim 11 or 12, **characterised in that** the sealing element (33) has or consists of an elastic tube which for sealing is pumped up by means of a gas, preferably compressed air.

## Revendications

1. Dispositif destiné à obtenir une greffe en couches de la cornée de l'œil humain à partir d'un disque scléro-cornéen par injection de liquide ou de gaz, comprenant
- un dispositif de retenue (12) pour le disque sclérotique (11) ; **caractérisé par** :
- au moins une zone d'accès (22) formée au niveau du dispositif de retenue (12), par rapport au disque sclérotique (11) fixé dans celui-ci, dans laquelle une aiguille creuse (26) peut être introduite dans la région périphérique de la cornée (18) à travers une ouverture dans le disque sclérotique (11) entre la membrane de Descemet et le stroma cornéen ; et
- un élément d'étanchéité (33) élastique pouvant être placé au moins dans la zone d'accès (22) contre le dispositif de retenue (12) ou le disque sclérotique (11) qui y est contenu pour enserrer de manière étanche le disque sclérotique (11) et l'aiguille creuse (26) introduite via l'ouverture au moins au niveau de la zone d'accès (22).

2. Dispositif selon la revendication 1, **caractérisé en ce que** le dispositif de retenue (12) présente un premier élément de serrage (20) de forme annulaire et au moins un deuxième élément de serrage (21) interagissant avec le premier élément de serrage (20), entre lesdits éléments de serrage (20, 21) le disque sclérotique (11) peut être enserré au niveau d'une région de retenue externe de préférence de forme annulaire (19).

3. Dispositif selon la revendication 1 ou 2, **caractérisé en ce que** le dispositif de retenue (12) présente un agencement de dépression (27), au moyen duquel le disque sclérotique (11) peut être aspiré au niveau d'une surface de retenue (17, 20).

4. Dispositif selon la revendication 2 ou 3, **caractérisé en ce que** le premier élément de serrage (20) est formé dans la région périphérique d'une cavité ressemblant quelque peu à une calotte (17) réalisée lors d'une intervention (16) pratiquée sur le disque sclérotique (11).

5. Dispositif selon l'une quelconque des revendications 2 à 4, **caractérisé en ce que** le deuxième élément de serrage (21) est formé en tant que bague de serrage ou bouton-poussoir (23) avec rondelle de pression insérée (24), dans lequel une interruption formant la zone d'accès (22) est prévue sur sa périphérie.

6. Dispositif selon l'une quelconque des revendications 2 à 5, **caractérisé en ce que** le premier et le deuxième éléments de serrage sont reliés entre eux de manière à pouvoir pivoter et à pouvoir être verrouillés à l'état de serrage.

7. Dispositif selon l'une quelconque des revendications 1 à 6, **caractérisé en ce que** l'élément d'étanchéité (33) se compose sensiblement d'un matériau compressible et/ou d'un élément tubulaire gonflable.

8. Dispositif selon l'une quelconque des revendications 1 à 7, **caractérisé par** un élément de pression, qui est relié en formant une distance à au moins l'un des premier et deuxième éléments de serrage et qui sert de butée à l'élément d'étanchéité.

9. Dispositif selon l'une quelconque des revendications 1 à 8, **caractérisé par** un élément de lame (35) disposé en particulier de manière concentrique autour du(des) élément(s) de serrage (20; 21) de forme annulaire, qui peut être amené en contact de coupe avec la membrane de Descemet du disque sclérotique serré (11).

10. Dispositif selon la revendication 9, **caractérisé en ce que** l'élément de lame (35) est conçu en tant que lame de découpage adapté à la forme souhaité de la greffe en couches à obtenir, de préférence de forme annulaire.

11. Procédé destiné à obtenir une greffe en couches de la cornée de l'œil humain à partir d'un disque scléro-cornéen d'un donneur décédé par injection de liquide ou de gaz, en utilisant un dispositif selon l'une quelconque des revendications 1 à 10, comprenant les étapes consistant à :
- maintenir le disque sclérotique (11) provenant d'un œil de donneur préparé au préalable au moyen d'un dispositif de retenue (12) de préférence au niveau d'une région de retenue externe de forme annulaire (19) de sorte qu'au niveau de ou à proximité du dispositif de retenue (12) au moins une zone d'accès (22) est formée dans le disque sclérotique serré ;
- pratiquer une ouverture dans le disque sclérotique au niveau de la zone d'accès (22) et introduire une aiguille creuse (26) entre la membrane de Descemet et le stroma cornéen dans le disque sclérotique (11) ;
- obturer l'ouverture par rapport à l'aiguille creuse (26) introduite dans celui-ci au moyen d'un élément d'étanchéité élastique (33), qui a été employé au moins au niveau de la zone d'accès (22) contre le dispositif de retenue ou le disque sclérotique qui y est contenu et enserrant hermétiquement l'aiguille creuse qui y a été introduite au niveau de la zone d'accès entre eux et le dispositif de retenue (12) ;
- introduire un liquide ou un gaz par l'aiguille creuse sous une pression positive appropriée entre la membrane de Descemet et le stroma cornéen dans le disque sclérotique (11) ; et
- sectionner pour libérer la membrane de Descemet séparée de la couche de stroma de la cornée.

12. Procédé selon la revendication 11, **caractérisé en ce que** la section pour libérer la membrane de Descemet s'effectue au moyen d'une lame de découpage de préférence de forme annulaire, qui est disposé sur le dispositif de préférence de manière concentrique au dispositif de retenue (12) et qui lors de l'introduction de liquide ou de gaz dans le disque sclérotique vient en contact avec la membrane de Descemet détachée de la couche de stroma.

13. Procédé selon la revendication 11 ou 12, **caractérisé en ce que** l'élément d'étanchéité (33) présente un tuyau élastique ou se compose d'un élément qui est gonflé pour assurer l'étanchéité au moyen d'un gaz, de préférence d'air comprimé.
